(19) Europäisches Patentamt
European Patent Office
Office européen des brevets

(11) EP 3 854 403 B1

(12) EUROPEAN PATENT SPECIFICATION

(45) Date of publication and mention of the grant of the patent:
**18.06.2025 Bulletin 2025/25**

(21) Application number: **20202229.9**

(22) Date of filing: **16.10.2020**

(51) International Patent Classification (IPC):
***A61K 31/685*** *(2006.01)* ***A61P 31/14*** *(2006.01)*
***A61P 11/00*** *(2006.01)*

(52) Cooperative Patent Classification (CPC):
**A61K 31/685; A61P 11/00; A61P 31/14**

# (54) USE OF SUBSTITUTED AMINOPROPIONATE COMPOUNDS IN TREATMENT OF SARS-COV-2 INFECTION

VERWENDUNG VON SUBSTITUIERTEN AMINOPROPIONSÄUREVERBINDUNGEN ZUR BEHANDLUNG VON SARS-COV-2-INFEKTION

UTILISATION DE COMPOSÉS D'AMINOPROPIONATE SUBSTITUÉS DANS LE TRAITEMENT DE L'INFECTION SARS-COV-2

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **21.01.2020 CN 202010071087**

(43) Date of publication of application:
**28.07.2021 Bulletin 2021/30**

(73) Proprietor: **Academy of Military Medical Sciences**
**Beijing 100850 (CN)**

(72) Inventors:
- **ZHONG, Wu**
  **BEIJING, 100850 (CN)**
- **CAO, Ruiyuan**
  **BEIJING, 100850 (CN)**
- **XIAO, Gengfu**
  **BEIJING, 100850 (CN)**
- **HU, Zhihong**
  **BEIJING, 100850 (CN)**
- **WANG, Manli**
  **BEIJING, 100850 (CN)**
- **ZHANG, Leike**
  **BEIJING, 100850 (CN)**
- **LI, Wei**
  **BEIJING, 100850 (CN)**
- **LI, Yuexiang**
  **BEIJING, 100850 (CN)**
- **ZHAO, Lei**
  **BEIJING, 100850 (CN)**
- **FAN, Shiyong**
  **BEIJING, 100850 (CN)**
- **LI, Song**
  **BEIJING, 100850 (CN)**

(74) Representative: **Lavoix**
**Bayerstraße 83**
**80335 München (DE)**

(56) References cited:
**WO-A1-2017/049060**

- **ANONYMOUS: "Gillings research on broad-spectrum antiviral could aid public health response to coronavirus outbreaks - UNC Gillings School of Global Public Health", 10 January 2020 (2020-01-10), XP055782994, Retrieved from the Internet <URL:https://sph.unc.edu/sph-news/gillings-research-on-broad-spectrum-antiviral-could-aid-public-health-response-to-coronavirus-outbreaks/> [retrieved on 20210308]**
- **NATURE PORTFOLIO MICROBIOLOGY COMMUNITY: "Preparing for future pandemics, today with broad-spectrum antivirals | Nature Portfolio Microbiology Community", 10 January 2020 (2020-01-10), XP055783036, Retrieved from the Internet <URL:https://naturemicrobiologycommunity.nature.com/posts/58125-preparing-for-future-pandemics-today-with-broad-spectrum-antivirals> [retrieved on 20210308]**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

- **SHEAHAN TIMOTHY P. ET AL: "Broad-spectrum antiviral GS-5734 inhibits both epidemic and zoonotic coronaviruses", SCIENCE TRANSLATIONAL MEDICINE, vol. 9, no. 396, 28 June 2017 (2017-06-28), US, pages eaal3653, XP055783008, ISSN: 1946-6234, DOI: 10.1126/ scitranslmed.aal3653**
- **MARIA L. AGOSTINI ET AL: "Coronavirus Susceptibility to the Antiviral Remdesivir (GS5734) Is Mediated by the Viral Polymerase and the Proofreading Exoribonuclease", MBIO, vol. 9, no. 2, 6 March 2018 (2018-03-06), pages 1 - 15, XP055677700, DOI: 10.1128/mBio.00221-18**
- **ALLISON L. TOTURA ET AL: "Broad-spectrum coronavirus antiviral drug discovery", EXPERT OPINION ON DRUG DISCOVERY, vol. 14, no. 4, 8 March 2019 (2019-03-08), London, GB, pages 397 - 412, XP055677701, ISSN: 1746-0441, DOI: 10.1080/17460441.2019.1581171**


Remarks:

The complete document including Reference Table(s) and the Sequence Listing(s) can be downloaded from the EPO website

## Description

The present application is based on and claims the benefit of priority from Chinese application No. 202010071087.7, filed on January 21, 2020.

## Technical Field

The present application relates to use of a substituted aminopropionate compound represented by the following Formula I, a geometric isomer, a pharmaceutically acceptable salt, a solvate and/or a hydrate thereof, and a pharmaceutical composition comprising the above compound for use in treating a SARS-CoV-2 infection.

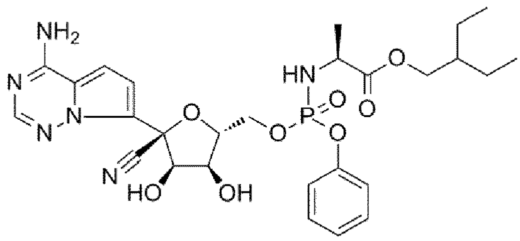

I

## Background Art

The substituted aminopropionate compound (Compound represented by Formula I), with chemical name of 2-ethylbutyl (2S)-2-[[[(2R,3S,4R,5R)-5-(4-aminopyrrolo[2,1-f][1,2,4]triazin-7-yl)-5-cyano-3,4-dihydroxyoxolan-2-yl]methoxy-phenoxyphosphoryl]amino]propanoate, also known as Remdesivir (GS-5734), is a viral RNA polymerase inhibitor, and its active form *in vivo* is a nucleoside triphosphate (NTP) of the parent drug. The phase III clinical trial of the drug compound for clinical treatment of Ebola virus infection has been now completed, and has shown good therapeutic effect on Ebola virus infection.

Remdesivir has shown antiviral activity against various variants of EBOV and other filoviruses in cell-based assays. In the Ebola virus-infected rhesus monkey model, even if the treatment started three days after virus exposure (systemic viral RNA was detected in two of the six treated animals), the intravenous administration of 10 mg/kg of GS-5734 per day for consecutive 12 days could significantly inhibit the replication of EBOV, protect 100% EBOV-infected animals from death, and improve clinical signs and pathophysiological markers. In addition to Ebola virus, GS-5734 also has a broad-spectrum antiviral activity to viruses such as Nipah virus, Middle East Respiratory Syndrome Coronavirus (MERS-CoV), Marburg virus, etc.

The 2019 novel Coronavirus (2019-nCoV) is a new coronavirus strain that has never been found in humans before. On February 11, 2020, the International Committee on Taxonomy Viruses (ICTV) announced that the official name of 2019 novel Coronavirus (2019-nCoV) is severe acute respiratory syndrome coronavirus 2 (SARS-CoV-2). On the same day, the World Health Organization (WHO) announced that the official name of the disease caused by this virus is COVID-19. The symptoms of SARS-CoV-2 virus infection are mainly pneumonia, and can be divided into simple infection, mild pneumonia, severe pneumonia, acute respiratory distress syndrome, sepsis, septic shock and so on according to the severity of disease. Patients with simple infection may have non-specific symptoms, such as fever, cough, sore throat, nasal congestion, fatigue, headache, muscle pain or discomfort, and the elderly people and immunosuppressed people may have atypical symptoms. Patients with mild pneumonia mainly have cough, dyspnea and polypnea. Severe pneumonia can be seen in adolescents, adults or children, and the main symptoms of which include increased breathing frequency, severe respiratory failure or dyspnea, central cyanosis, drowsiness, unconsciousness or convulsion, gasp, etc. The lung images of acute respiratory distress syndrome are bilateral ground glass shadows, which cannot be completely explained by effusion, lobar exudation or atelectasis or lung mass shadows, and the main symptom of which is pulmonary edema. Patients with sepsis often have fatal organ dysfunction, and the most critical patients are those with septic shock, and they may have a high probability of death.

Anonymous: Gillings research on broad-spectrum antiviral could aid public health response to coronavirus outbreaks - UNC Gillings School of Global Public Health, 10 January 2020, discloses that remdesivir is effective against coronaviruses like SARS-CoV. Nature Portfolio Microbiology Community, "Preparing for future pandemics, today with broad-spectrum antivirals, Nature Portfolio Microbiology Community, 10 January 2020, discloses that remdesivir may be effective against the coronavirus. Sheahan T. et al, Science Translational Medicine, 2017, 9(396), eaal3653, relates to

broad-spectrum antiviral GS-5734 inhibiting both epidemic and zoonontic coronaviruses. Agostini ML et al, MBIO, 2018, 9(2), 1-15 relates to coronavirus susceptibility to remdesivir being mediated by the viral polymerase and the proofreading exoribonuclease. Totura AL et al., Expert Opinion on Drug Discovery, 2019, 14(4), 397-412 relates to the discovery of broad-spectrum coronavirus antiviral drugs.

At present, the SARS-CoV-2 virus infection is mainly treated with supportive therapy in clinic, and no antiviral drug is available.

**Contents of the application**

The purpose of the present application is to find a drug with antiviral activity against SARS-CoV-2, which can be used for the treatment of a related disease caused by the infection thereof. Through creative research, it is found in the present application that the compound represented by Formula I has the function of inhibiting the replication of SARS-CoV-2 and has a good potential therapeutic effect in the treatment of a disease caused by SARS-CoV-2.

The present application relates to the compound represented by Formula I, a geometric isomer, a pharmaceutically acceptable salt and/or a solvate or a hydrate thereof,

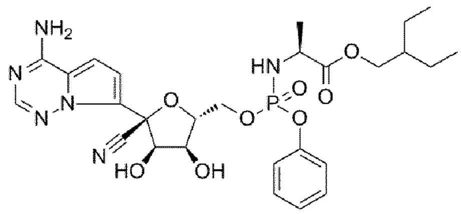

I

for use in treating a disease or an infection caused by a SARS-CoV-2.

In some embodiments, the pharmaceutically acceptable salts of the compound represented by Formula I described herein include inorganic or organic acid salts and inorganic or organic base salts thereof. The present application relates to all forms of the above salts, including but not limited to: sodium salt, potassium salt, calcium salt, lithium salt, meglumine salt, hydrochloride salt, hydrobromide salt, hydroiodide salt, nitrate salt, sulfate, hydrogen sulfate, phosphate, hydrogen phosphate, acetate, propionate, butyrate, oxalate, pivalate, adipate, alginate, lactate, citrate, tartrate, succinate, maleate, fumarate, picrate, aspartate, gluconate, benzoate, methanesulfonate, ethanesulfonate, benzenesulfonate, p-toluenesulfonate and embonate and so on.

The compound represented by Formula I can inhibit SARS-CoV-2 virus replication in cells and reduce the nucleic acid load of SARS-CoV-2 virus in cell culture.

After creative invention research, the inventors of the present application have discovered new features of the compound represented by Formula I in cells: the compound represented by Formula I can reduce the viral nucleic acid load in cells infected by SARS-CoV-2 at micromolar concentration level.

The present application thus relates to the compound represented by Formula I, a geometric isomer, a pharmaceutically acceptable salt, a solvate and/or a hydrate thereof for use for treating a disease or an infection (e.g., a respiratory disease (e.g., a simple infection (such as fever, cough and sore throat), pneumonia, acute respiratory infection, severe acute respiratory infection (SARI), hypoxic respiratory failure and acute respiratory distress syndrome, sepsis and septic shock, etc.)) caused by a SARS-CoV-2,

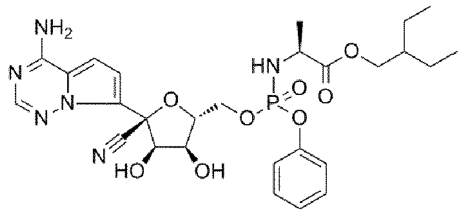

I.

The present application also relates to a pharmaceutical composition, which comprises the compound represented by Formula I, a geometric isomer, a pharmaceutically acceptable salt, a solvate and/or a hydrate thereof for use in

treating a disease or an infection caused by a SARS-CoV-2.

Preferably, the pharmaceutical composition further comprises a pharmaceutically acceptable carrier or excipient. Specifically, the pharmaceutical composition is a solid preparation, an injection, an external preparation, a spray, a liquid preparation, or a compound preparation.

In some embodiments, the pharmaceutical composition comprises an effective amount of the compound represented by formula I, a geometric isomer, a pharmaceutically acceptable salt, a solvate and/or a hydrate thereof.

The present application thus relates to the pharmaceutical composition comprising the compound represented by Formula I, a geometric isomer, a pharmaceutically acceptable salt, a solvate and/or a hydrate thereof for use for treating a disease or an infection caused by a SARS-CoV-2 (e.g., a respiratory disease (e.g., a simple infection (such as fever, cough and sore throat), pneumonia, acute respiratory infection, severe acute respiratory infection (SARI), hypoxic respiratory failure and acute respiratory distress syndrome, sepsis and septic shock, etc.)).

The present application also relates to a pharmaceutical composition comprising the compound of Formula I, a geometric isomer, a pharmaceutically acceptable salt, a solvate and/or a hydrate thereof, or a compound represented by Formula I, a geometric isomer, a pharmaceutically acceptable salt, a solvate and/or a hydrate thereof for use in a method for treating and/or preventing a disease in a mammal in need, comprising administering to the mammal in need a therapeutically and/or prophylactically effective amount of the pharmaceutical composition comprising the compound of Formula I, a geometric isomer, a pharmaceutically acceptable salt, a solvate and/or a hydrate thereof or a compound of Formula I, a geometric isomer, a pharmaceutically acceptable salt, a solvate and/or a hydrate thereof, wherein the disease includes a disease caused by a SARS-CoV-2, such as a viral infectious disease caused by a SARS-CoV-2 (e.g., respiratory disease, including simple infection (such as fever, cough and sore throat, etc.), pneumonia, acute respiratory infection, severe acute respiratory infection (SARI), hypoxic respiratory failure and acute respiratory distress syndrome, sepsis and septic shock, etc.).

The present application accordingly relates to the compound represented by Formula I, a geometric isomer, a pharmaceutically acceptable salt, a solvate and/or a hydrate thereof, for use in treating a disease or an infection (e.g., a respiratory disease (e.g., a simple infection such as fever, cough and sore throat), pneumonia, acute respiratory infection, severe acute respiratory infection (SARI), hypoxic respiratory failure and acute respiratory distress syndrome, sepsis and septic shock, etc.) caused by a SARS-CoV-2.

The present application accordingly relates to the pharmaceutical composition comprising the compound represented by Formula I, a geometric isomer, a pharmaceutically acceptable salt, a solvate and/or a hydrate thereof, for use in treating a disease or an infection (e.g., a respiratory disease (e.g., a simple infection (such as fever, cough and sore throat), pneumonia, acute respiratory infection, severe acute respiratory infection (SARI), hypoxic respiratory failure and acute respiratory distress syndrome, sepsis and septic shock, etc.)) caused by a SARS-CoV-2.

In some embodiments, the disease caused by SARS-CoV-2 described in the present application includes but is not limited to respiratory disease, such as simple infection (such as fever, cough and sore throat), pneumonia, acute respiratory infection, severe acute respiratory infection (SARI), hypoxic respiratory failure and acute respiratory distress syndrome, sepsis and septic shock.

In some embodiments, the disease caused by SARS-CoV-2 described herein is COVID-19.

In the present application, the official name of the term "2019 novel Coronavirus (2019-nCoV)" is severe acute respiratory syndrome coronavirus 2 (SARS-CoV-2).

In the present application, the official name of the term "disease caused by 2019 novel Coronavirus (2019-nCoV)" is COVID-19.

The pharmaceutical composition described in the present application can be prepared into various forms according to different administration routes.

According to the present application, the pharmaceutical composition can be administered in any one of the following routes: oral administration, spray inhalation, rectal administration, nasal administration, buccal administration, vaginal administration, topical administration, parenteral administration such as subcutaneous, intravenous, intramuscular, intraperitoneal, intrathecal, intraventricular, intrasternal and intracranial injection or infusion, or administration with the help of an explant reservoir, wherein the preferred administration route is oral, intraperitoneal or intravenous.

When orally administered, the compound represented by Formula I, a geometric isomer, a pharmaceutically acceptable salt, a solvate and/or a hydrate thereof can be prepared into any form of orally acceptable preparation, including but not limited to a tablet, a capsule, an aqueous solution or an aqueous suspension. The carrier for use in a tablet generally includes lactose and corn starch, and a lubricant such as magnesium stearate can also be added. The diluent for use in a capsule generally includes lactose and dry corn starch. The aqueous suspension is usually used by mixing an active ingredient with a suitable emulsifier and a suitable suspending agent. If necessary, a sweetener, a flavoring agent or a coloring agent can also be added to the above-mentioned forms of oral preparation.

When rectally administered, the compound represented by Formula I, a geometric isomer, a pharmaceutically acceptable salt, a solvate, and/or a hydrate thereof can generally be prepared in a form of suppository, which is prepared by mixing the drug with a suitable non-irritating excipient. The excipient is present in solid state at room temperature, but melts

at the rectal temperature to release the drug. Such excipient includes cocoa butter, beeswax and polyethylene glycol.

When topically administered, especially for treatment of easily accessible affected-surface or organ, such as eye, skin, or lower intestinal neurological disease by topical application, the compound represented by Formula I, a geometric isomer, a pharmaceutically acceptable salt, a solvate and/or a hydrate thereof can be prepared in various forms of topical preparations according to different affected-surfaces or organs, the specific instructions are as follows:

When topically administered to eye, the compound represented by Formula I, a geometric isomer, a pharmaceutically acceptable salt, a solvate and/or a hydrate thereof can be formulated into a preparation form such as micronized suspension or solution, the carrier used is isotonic sterile saline with a certain pH, and a preservative such as benzyl chloride alkoxide may or may not be added. In addition, for administration to eye, the compound can also be prepared in a form of ointment such as vaseline ointment.

When topically administered to skin, the compound represented by Formula I, a geometric isomer, a pharmaceutically acceptable salts, a solvate and/or a hydrate thereof can be prepared into a suitable form such as an ointment, a lotion or a cream, in which the active ingredient is suspended or dissolved in one or more carriers. The carrier for use in an ointment includes, but is not limited to: mineral oil, liquid petrolatum, white petrolatum, propylene glycol, polyethylene oxide, polypropylene oxide, emulsifying wax, and water. The carrier for use in a lotion or a cream includes, but is not limited to: mineral oil, sorbitan monostearate, Tween-60, cetyl ester wax, hexadecenyl aryl alcohol, 2-octyldodecanol, benzyl alcohol and water.

When topically administered to lower intestinal tract, the compound represented by Formula I, a geometric isomer, a pharmaceutically acceptable salt, a solvate and/or a hydrate thereof can be prepared into a form such as rectal suppository as described above or a suitable enema preparation form, in addition, a topical transdermal patch can also be used.

The compound represented by Formula I, a geometric isomer, a pharmaceutically acceptable salt, a solvate and/or a hydrate thereof can also be administered in a preparation form of sterile injection, including sterile injectable aqueous solution or oil suspension, or sterile injectable solutions, wherein the usable carrier and solvent includes water, Ringer's solution and isotonic sodium chloride solution. In addition, a sterilized non-volatile oil such as monoglyceride or diglyceride can also be used as solvent or suspension media.

The drugs of the above various preparation forms can be prepared according to conventional methods in the pharmaceutical field.

In the present application, the term "therapeutically effective amount" or "prophylactically effective amount" refers to an amount that is sufficient to treat or prevent a patient's disease but is sufficiently low to avoid serious side effects (at a reasonable benefit/risk ratio) within a reasonable medical judgment. The therapeutically effective amount of the compound will change according to the factors such as the selected specific compound (e.g., considering the efficacy, effectiveness, and half-life of compound), the selected administration route, the treated disease, the severity of the treated disease, the patient's age, size, weight and physical disease, medical history, duration of treatment, nature of concurrent therapy, desired therapeutic effect, etc., but can still be routinely determined by those skilled in the art.

In addition, it should be noted that the specific dosage and method of using the compound represented by Formula I, a geometric isomer, a pharmaceutically acceptable salt, a solvate and/or a hydrate thereof for different patients depends on many factors, including the patient's age, weight, gender, natural health status, nutritional status, active strength of drug, administration time, metabolic rate, severity of disease, and subjective judgment of physician. Herein it is preferred to use a dosage between 0.001-1000 mg/kg body weight/day.

## Brief Description of the Drawings

Figure 1 shows that Remdesivir can effectively reduce the viral nucleic acid load in Vero E6 cells infected by SARS-CoV-2 virus. In Figure 1, (a) shows that Remdesivir can reduce the viral RNA load in the cells 48 hours after the cells were infected by SARS-CoV-2 virus, and the drug concentration of 33 μM can reduce the viral nucleic acid load by an order of magnitude. The ordinate is the copy number of viral RNA in the sample, and the abscissa is the drug concentration; (b) shows that the test cells were treated by Remdesivir at the test concentration for 48 hours, and no cytotoxicity was observed. The ordinate is the percentage of cell viability relative to the vehicle control group (only cells, no drug added), and the abscissa is the drug concentration.

## Specific Models for Carrying Out the Application

The following examples are illustrative preferred embodiments of the present application.

Example 1: Experiment of Remdesivir in reduction of viral nucleic acid load of cells infected by SARS-CoV-2 virus

(1) Drug treatment of virus-infected cells

Vero E6 cells (purchased from ATCC, Catalog No. 1586) were placed into a 24-well plate and incubated for 24 hours, then virus infection was carried out, specifically, SARS-CoV-2 (2019-nCoV) virus (nCoV-2019BetaCoV/Wuhan/-WIV04/2019 strain, provided by Wuhan Institute of Virology, Chinese Academy of Sciences) was diluted with 2% cell maintenance solution (formulation: FBS (purchased from Gibco, Catalog No.: 16000044) was added to MEM (purchased from Gibco, Article No: 10370021) by a volume ratio of 2%, thereby obtaining the 2% cell maintenance solution) to corresponding concentration, and then added to the 24-well plate so that each well contained a viral load of 100$TCID_{50}$. Next, the Remdesivir (purchased from MedChemExpress, Catalog No.: HY-104077) was diluted with 2% cell maintenance solution to the corresponding concentrations and added to corresponding wells, so that the final drug concentrations were 100 μM, 33 μM, 11 μM, 3.7 μM, 1.23μM, 0.41μM, 0.14μM, respectively, then the plate was put in 37°C, 5% $CO_2$ incubator and continuously cultured for 48h; and the vehicle control group was added with only 2% cell maintenance solution without any test drug.

(2) RNA extraction

The RNA extraction kit was purchased from Qiagen, Catalog No.: 74106. The consumptive materials (spin column, RNase-free 2ml collection tube, etc.) and reagents (RLT, RW1, RPE, RNase-free water, etc.) involved in the following RNA extraction steps were all parts of the kit. The following extraction steps were all recommended by the kit instructions.

1) 100 μL of the supernatant was taken from the test plate, added to a nuclease-free EP tube, then added with 350 μL of Buffer RLT, mixed with a transfer liquid gun to make it fully lysed, and centrifuged to take the supernatant;

2) the supernatant obtained in step 1) was added with an equal volume of 70% ethanol and mixed well;

3) the mixed solution obtained in step 2) above was transferred to a RNase-free spin column, centrifuged at 12000 rpm for 15s, and the waste liquid was discarded;

4) 700μL of Buffer RW1 was added to the spin column, then centrifugation was carried out at 12000 rpm for 15s to clean the spin column, and the waste liquid was discarded;

5) 500μL of Buffer RPE was added to the spin column, then centrifugation was carried out at 12000 rpm for 15s to clean the spin column, and the waste liquid was discarded;

6) 500μL of Buffer RPE was added to the spin column, then centrifugation was carried out at 12000 rpm for 2min to clean the spin column, and the waste liquid was discarded;

7) the spin column was placed in a new RNase-free 2 ml collection tube, and centrifugation was carried out at 12000 rpm for 1 min to dry the spin column, and then the entire spin column was transferred to the 1.5 ml collection tube of step 8);

8) the spin column dried in step 7) was placed in a new 1.5ml collection tube, added with 30μl of RNase-free water, and centrifuged at 12000 rpm for 2min, the obtained eluent contained the corresponding RNA, was added with RNase inhibitor (purchased from NEB, Catalog No.: M0314L), and detected with Nano Drop (purchased from Thermo scientific, Nano Drop One) to determine each RNA concentration.

(3) RNA reverse transcription

In the experiment, the reverse transcription kit (PrimeScript™ RT reagent Kit with gDNA Eraser, Catalog No. RR047Q) produced by TaKaRa Company was used for RNA reverse transcription. The steps were as follows.

① gDNA removal: RNA samples from each experimental group were collected, and 1 μg thereof was taken and subjected to reverse transcription. First, 2 μl of 5× gDNA Eraser Buffer was added to the RNA sample of each experimental group, the reaction system was supplemented with RNase Free water to 10 μl, mixed well, and subjected to 42 °C water bath for 2 min to remove the gDNA that might exist in the sample;

② Reverse transcription: the sample obtained in ① was added with appropriate amounts of enzyme, primer Mix and

reaction buffer, supplemented with RNase Free water to a volume of 20 μl, reacted under 37°C water bath for 15 min, then put in 85°C water bath for 5 sec, thereby obtaining cDNA via transcription.

(4) Real-time PCR

Fluorescence quantitative PCR was used to detect the copy number per ml of the original virus solution.

The reaction system was mixed using TB Green Premix (Takara, Cat#RR820A), and the amplification reaction and reading were carried out with StepOne Plus Real-time PCR instrument (brand: ABI). The copy number contained in per ml of the original virus solution was calculated. The steps were as follows:

① Establishment of standard product: the plasmid pMT-RBD (the plasmid was provided by Wuhan Institute of Virology, Chinese Academy of Sciences) was diluted to $5\times10^8$ copies/μL, $5\times10^7$ copies/μL, $5\times10^6$ copies/μL, $5\times10^5$ copies/μL, $5\times10^4$ copies/μL, $5\times10^3$ copies/μL, $5\times10^2$ copies/μL. 2 μL standard or cDNA template was taken for qPCR reaction.

② The sequences of primers used in the experiment were as follows (all indicated in 5'-3' direction):

RBD-qF: CAATGGTTTAACAGGCACAGG

RBD-qR: CTCAAGTGTCTGTGGATCACG

③ The reaction procedure was as follows:

Pre-denaturation: 95 °C for 5 minutes;

Cycle parameters: 95 °C for 15 seconds, 54 °C for 15 seconds, 72 °C for 30 seconds, for a total of 40 cycles.

(5) Cytotoxicity test of drug

The detection of the drug cytotoxicity was performed using CCK-8 kit (Beoytime). Specific steps were as follows:

① $1\times10^4$ Vero E6 (ATCC) cells were placed in a 96-well plate and incubated at 37°C for 8 hours.

② The drug was diluted with DMSO to an appropriate concentration of mother liquor, and then diluted with MEM medium (purchased from Gibco, Catalog No. 10370021) containing 2% FBS (purchased from Gibco, Catalog No. 16000044) to the same concentration as that for the drug treatment. The original medium in the 96-well plate was discarded, 100 μL of drug-containing MEM medium was added to the cells, and three replicate wells were prepared for each concentration. Vehicle control (DMSO and medium were added to the cell wells, without adding drug) and blank control (DMSO and medium were added to the wells, without cells) were set up. After the drug was added, the cells were cultured at 37°C for 48 hours.

③ 20 μL of CCK-8 solution (Beoytime) was added to the well to be tested, mixed gently, without generating bubbles, and continuously incubated at 37 °C for 2 hours. $OD_{450}$ was read on a microplate reader (purchased from Molecular Devices, Model: SpectraMax M5), and cell viability was calculated:

$$\text{Cell activity (\%)} = (A_{(\text{drug treatment group})} - A_{(\text{blank control})}) / (A_{(\text{vehicle control})} - A_{(\text{blank control})}) \times 100\%$$

wherein A was the reading of the microplate reader.

(6) Experimental results

The results of the virus proliferation inhibition experiment showed that the test compound at concentrations of 100 μM, 33 μM, 11.1 μM and 3.7 μM could effectively inhibit the replication of the SARS-CoV-2 virus genome in the infected supernatant (Table 1 and Figure 1)

Table 1. *In vitro* antiviral test of the test compound (Remdesivir)

| Concentration (μM) | 100 | 33.33 | 11.11 | 3.70 | 1.23 | 0.41 | 0.14 | Vehicle |
|---|---|---|---|---|---|---|---|---|
| Virus genome copy number | 9010444. 5 ±22822 39.02 | 1242973 2 ±18125 81.22 | 1630851 6.5±1475 181.58 | 2028751 2.5±1308 4251.56 | 8792736 50.5 ±189 609425.3 2 | 1751863 138.5 ±11 1506981 2.46 | 6120820 311.5 ±28 8672855 1.13 | 5108150 26 ±1805 18414.22 |

The cytotoxicity test results showed that the treatment of the test compound (Remdesivir) did not change the cell viability at all test concentrations, that was, the test compound had no toxic effect on the cells at all concentrations (Table 2 and Figure 1).

Table 2. Cytotoxicity test results of the test compound (Remdesivir)

| Concentration (μM) | 100 | 33.33 | 11.11 | 3.70 | 1.23 | 0.41 | 0.14 | Vehicle |
|---|---|---|---|---|---|---|---|---|
| Cell viability (% of vehicle control) | 97.70± 0.76 | 99.06± 0.41 | 97.05± 1.12 | 97.40± 0.41 | 97.88± 1.12 | 100.76± 1.17 | 100.97± 2.61 | 100.19± 2.62 |

## Claims

1. A compound represented by Formula I, a geometric isomer, a pharmaceutically acceptable salt, a solvate and/or a hydrate thereof,

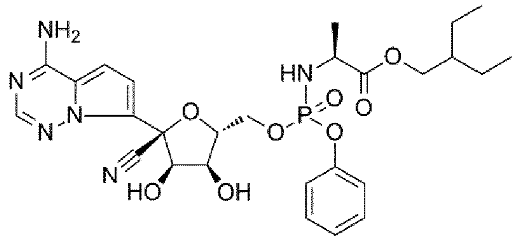

I

for use in treating a disease or an infection caused by a SARS-CoV-2.

2. The compound represented by Formula I, a geometric isomer, a pharmaceutically acceptable salt, a solvate and/or a hydrate thereof for use according to claim 1, wherein the disease caused by a SARS-CoV-2 is a respiratory disease.

3. The compound represented by Formula I, a geometric isomer, a pharmaceutically acceptable salt, a solvate and/or a hydrate thereof for use according to claim 1, wherein the disease caused by a SARS-CoV-2 is simple infection, pneumonia, acute respiratory tract infection, severe acute respiratory infection (SARI), hypoxic respiratory failure, acute respiratory distress syndrome, sepsis or septic shock.

4. The compound represented by Formula I, a geometric isomer, a pharmaceutically acceptable salt, a solvate and/or a hydrate thereof for use according to claim 3, wherein the simple infection is fever, cough and/or sore throat.

5. The compound, a geometric isomer, a pharmaceutically acceptable salt, a solvate and/or a hydrate thereof for use according to claim 1, wherein the disease caused by a SARS-CoV-2 is COVID-19.

6. A pharmaceutical composition for use in treating a disease or an infection caused by a SARS-CoV-2, wherein the pharmaceutical composition comprises a compound represented by Formula I, a geometric isomer, a pharmaceutically acceptable salt, a solvate and/or a hydrate thereof,

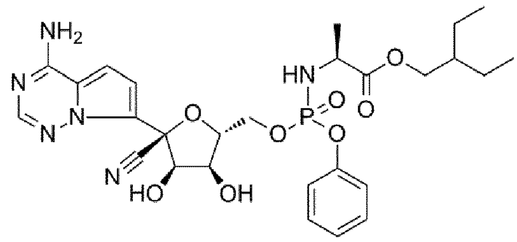

I.

7. The pharmaceutical composition for use according to claim 6, wherein the disease caused by a SARS-CoV-2 is a respiratory disease.

8. The pharmaceutical composition for use according to claim 6, wherein the disease caused by a SARS-CoV-2 is simple infection, pneumonia, acute respiratory tract infection, severe acute respiratory infection (SARI), hypoxic respiratory failure, acute respiratory distress syndrome, sepsis or septic shock.

9. The pharmaceutical composition for use according to claim 8, wherein the simple infection is fever, cough and/or sore throat.

10. The pharmaceutical composition for use according to any one of claims 6-9, wherein the pharmaceutical composition further comprises a pharmaceutically acceptable carrier or excipient,
preferably, wherein the pharmaceutical composition is a solid preparation, an injection, an external preparation, a spray, a liquid preparation, or a compound preparation.

11. The pharmaceutical composition for use according to claim 6, wherein the disease caused by a SARS-CoV-2 is COVID-19.

**Patentansprüche**

1. Verbindung, dargestellt durch Formel I, geometrisches Isomer, pharmazeutisch annehmbares Salz, Solvat und/oder Hydrat davon,

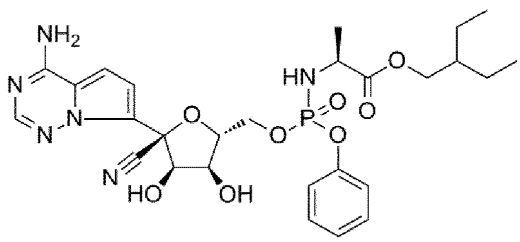

I

zur Verwendung beim Behandeln einer Erkrankung oder Infektion, die durch SARS-CoV-2 verursacht wird.

2. Verbindung, dargestellt durch Formel I, geometrisches Isomer, pharmazeutisch annehmbares Salz, Solvat und/oder Hydrat davon zur Verwendung nach Anspruch 1, wobei die durch SARS-CoV-2 verursachte Erkrankung eine Atemwegserkrankung ist.

3. Verbindung, dargestellt durch Formel I, geometrisches Isomer, pharmazeutisch annehmbares Salz, Solvat und/oder Hydrat davon zur Verwendung nach Anspruch 1, wobei die durch SARS-CoV-2 verursachte Erkrankung eine einfache Infektion, Lungenentzündung, akute Atemwegsinfektion, schwere akute Atemwegsinfektion (SARI), hypoxisches Atemversagen, akutes Atemnotsyndrom, Sepsis oder septischer Schock ist.

4. Verbindung, dargestellt durch Formel I, geometrisches Isomer, pharmazeutisch annehmbares Salz, Solvat und/oder Hydrat davon zur Verwendung nach Anspruch 3, wobei die einfache Infektion Fieber, Husten und/oder Halsentzündung ist.

5. Verbindung, dargestellt durch Formel I, geometrisches Isomer, pharmazeutisch annehmbares Salz, Solvat und/oder Hydrat davon zur Verwendung nach Anspruch 1, wobei die durch SARS-CoV-2 verursachte Erkrankung COVID-19 ist.

6. Pharmazeutische Zusammensetzung zur Verwendung beim Behandeln einer durch SARS-CoV-2 verursachten Erkrankung oder Infektion, wobei die pharmazeutische Zusammensetzung eine durch Formel I dargestellte Verbindung, ein geometrisches Isomer, ein pharmazeutisch annehmbares Salz, ein Solvat und/oder ein Hydrat davon umfasst,

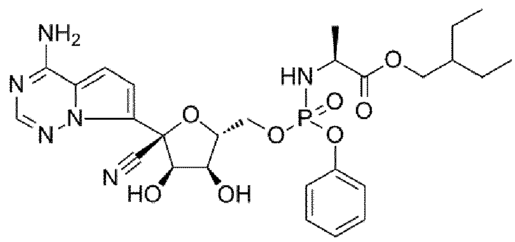

I.

7. Pharmazeutische Zusammensetzung zur Verwendung nach Anspruch 6, wobei die durch SARS-CoV-2 verursachte Erkrankung eine Atemwegserkrankung ist.

8. Pharmazeutische Zusammensetzung zur Verwendung nach Anspruch 6, wobei die durch SARS-CoV-2 verursachte Erkrankung eine einfache Infektion, Lungenentzündung, akute Atemwegsinfektion, schwere akute Atemwegsinfektion (SARI), hypoxisches Atemversagen, akutes Atemnotsyndrom, Sepsis oder septischer Schock ist.

9. Pharmazeutische Zusammensetzung zur Verwendung nach Anspruch 8, wobei die einfache Infektion Fieber, Husten und/oder Halsentzündung ist.

10. Pharmazeutische Zusammensetzung zur Verwendung nach einem der Ansprüche 6 bis 9, wobei die pharmazeutische Zusammensetzung ferner einen pharmazeutisch annehmbaren Träger oder Hilfsstoff umfasst, vorzugsweise, wobei die pharmazeutische Zusammensetzung eine feste Zubereitung, eine Injektion, eine externe Zubereitung, ein Spray, eine flüssige Zubereitung oder eine zusammengesetzte Zubereitung ist.

11. Pharmazeutische Zusammensetzung zur Verwendung gemäß Anspruch 6, wobei die durch SARS-CoV-2 verursachte Erkrankung COVID-19 ist.

## Revendications

1. Composé représenté par la formule I, isomère géométrique, sel pharmaceutiquement acceptable, solvate et/ou hydrate de celui-ci,

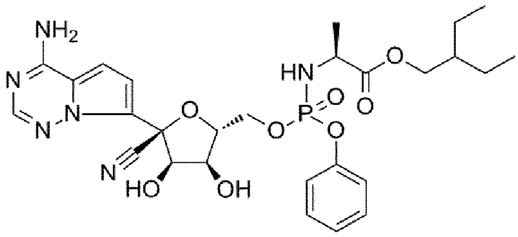

I

pour une utilisation dans le traitement d'une maladie ou d'une infection causée par un SARS-CoV-2.

2. Composé représenté par la formule I, isomère géométrique, sel pharmaceutiquement acceptable, solvate et/ou hydrate de celui-ci pour une utilisation selon la revendication 1, dans lequel la maladie causée par un SARS-CoV-2 est une maladie respiratoire.

3. Composé représenté par la formule I, isomère géométrique, sel pharmaceutiquement acceptable, solvate et/ou hydrate de celui-ci pour une utilisation selon la revendication 1, dans lequel la maladie causée par un SARS-CoV-2 est une simple infection, une pneumonie, une infection aiguë des voies respiratoires, une infection respiratoire aiguë sévère (SARI), une insuffisance respiratoire hypoxique, un syndrome de détresse respiratoire aiguë, une septicémie ou un choc septique.

4. Composé représenté par la formule I, isomère géométrique, sel pharmaceutiquement acceptable, solvate et/ou hydrate de celui-ci pour une utilisation selon la revendication 3, dans lequel l'infection simple est la fièvre, la toux et/ou le mal de gorge.

5. Composé, isomère géométrique, sel pharmaceutiquement acceptable, solvate et/ou hydrate de celui-ci pour une utilisation selon la revendication 1, dans lequel la maladie causée par un SARS-CoV-2 est le COVID-19.

6. Composition pharmaceutique pour une utilisation dans le traitement d'une maladie ou d'une infection causée par un SARS-CoV-2, dans laquelle la composition pharmaceutique comprend un composé représenté par la formule I, un isomère géométrique, un sel pharmaceutiquement acceptable, un solvate et/ou un hydrate de celui-ci,

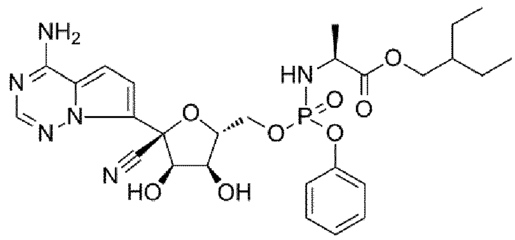

I.

7. Composition pharmaceutique pour une utilisation selon la revendication 6, dans laquelle la maladie causée par un SARS-CoV-2 est une maladie respiratoire.

8. Composition pharmaceutique pour une utilisation selon la revendication 6, dans laquelle la maladie causée par un SARS-CoV-2 est une simple infection, une pneumonie, une infection aiguë des voies respiratoires, une infection respiratoire aiguë sévère (SARI), une insuffisance respiratoire hypoxique, un syndrome de détresse respiratoire aiguë, une septicémie ou un choc septique.

9. Composition pharmaceutique pour une utilisation selon la revendication 8, dans laquelle l'infection simple est une fièvre, une toux et/ou un mal de gorge.

10. Composition pharmaceutique pour une utilisation selon l'une quelconque des revendications 6 à 9, dans laquelle la

composition pharmaceutique comprend en outre un support ou un excipient pharmaceutiquement acceptable, de préférence, dans laquelle la composition pharmaceutique est une préparation solide, une injection, une préparation externe, un spray, une préparation liquide ou une préparation composée.

**11.** Composition pharmaceutique pour une utilisation selon la revendication 6, dans laquelle la maladie causée par un SARS-CoV-2 est le COVID-19.

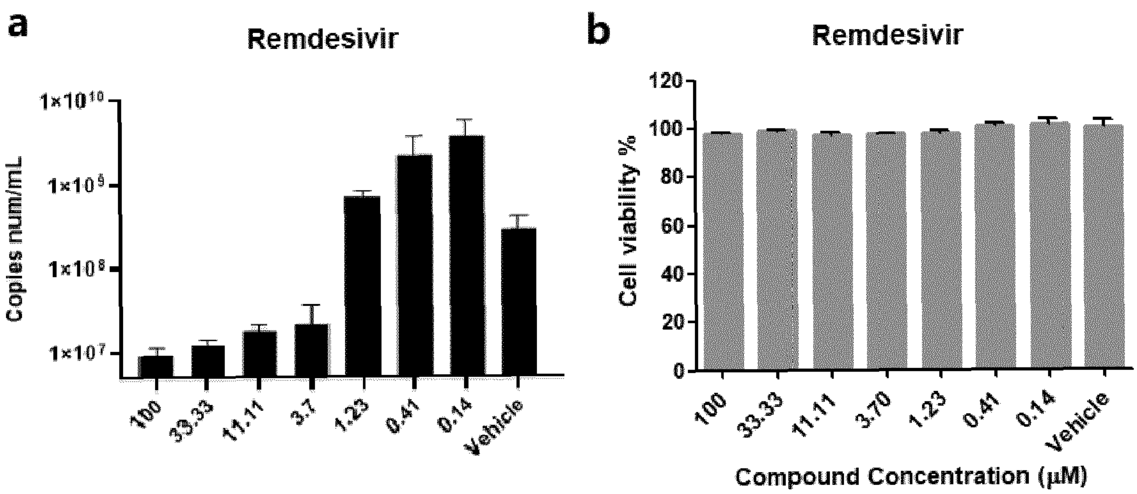
a
Remdesivir
1×10¹⁰
1×10⁹
1×10⁸
1×10⁷
Copies num/mL
100
33.33
11.11
3.7
1.23
0.41
0.14
Vehicle
b
Remdesivir
120
100
80
60
40
20
0
Cell viability %
100
33.33
11.11
3.70
1.23
0.41
0.14
Vehicle
Compound Concentration (μM)

Figure 1

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- CN 202010071087

### Non-patent literature cited in the description


- Preparing for future pandemics, today with broad-spectrum antivirals. *Nature Portfolio Microbiology Community*, 10 January 2020
- **SHEAHAN T. et al.** *Science Translational Medicine*, 2017, vol. 9 (396), eaal3653
- **AGOSTINI ML et al.** *MBIO*, 2018, vol. 9 (2), 1-15
- **TOTURA AL et al.** *Expert Opinion on Drug Discovery*, 2019, vol. 14 (4), 397-412